# EUROPEAN PATENT SPECIFICATION

(11) **EP 1 282 588 B1**
(45) Date of publication and mention of the grant of the patent: **05.01.2005**
(21) Application number: 01961421.3
(22) Date of filing: 11.05.2001
(51) Int. Cl.: C07C 37/055

(54) **PREPARATION OF HYDROXY COMPOUNDS**
HERSTELLUNG VON HYDROXYVERBINDUNGEN
PREPARATION DE COMPOSES HYDROXY

(30) Priority: 12.05.2000 NO 20002497
(43) Date of publication of application: 12.02.2003
(73) Proprietor: NORSK HYDRO ASA, 0240 Oslo (NO)
(72) Inventor: AASBO, Kari, N-3719 Skien (NO); GRANLI, Tom, N-3173 Vear (NO); BREIVIK, Harald, N-3942 Porsgrunn (NO)
(74) Representative: Wallace, Sheila Jane
(86) International application number: PCT/NO2001/000195
(87) International publication number: WO 2001/085658

(56) References cited:
- US-A- 2 697 732
- US-A- 4 473 713

## Description

This invention relates to a process for the preparation of certain hydroxy compounds and, in particular, certain aromatic hydroxy compounds.

It is well-known that ethers are generally unreactive compounds. Dialkyl ethers can be cleaved under strongly acidic conditions to form an alcohol by the intermediate formation of a dialkyloxonium salt using a strong acid such as hydrobromic or hydroiodic acid at reflux temperature. Similarly, aryl alkyl ethers are cleaved with hydrogen bromide or hydrogen iodide to yield a phenol and an alkyl halide at temperatures of 120-130°C. However, diaryl ethers, such as diphenyl ether, do not react with hydrogen iodide even at 200°C.

Surprisingly, it has now been found that certain ethers containing at least one aryl group can be converted to the corresponding hydroxy compound under mild conditions.

According to the present invention there is therefore provided a process for the preparation of a compound of the general formula

ArOH (I)

in which Ar represents a group of the general formula 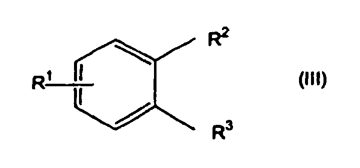 in which R¹ represents an electron-donating group, one of R² and R³ independently represents a hydrogen atom or an optionally substituted alkyl group and the other of R² and R³ represents an optionally substituted alkyl group, or R² and R³ together with the interjacent carbon atoms form an optionally substituted aryl group, which comprises reacting a compound of the general formula

ArOR (II)

or a compound which is capable of forming a compound of formula II in situ on reaction with a C₁₋₆ alkanoic acid in which the alkyl moiety is optionally substituted by a halogen atom, in which Ar is as defined above and R is an optionally substituted alkyl, aryl or aralkyl group, with an acid comprising at least one C₁₋₆ alkanoic acid in which the alkyl moiety is optionally substituted by a halogen atom.

Compounds which are readily convertible to compounds of formula II by reaction with an acid according to the process of the present invention can be referred to by the term "precursor. The term "precursor" therefore includes, for instance, compounds containing protecting groups which are easily removable by reaction with an acid to give a compound of formula II and compounds in which substituents on adjacent atoms of the aromatic ring together with the interjacent atoms form a ring which can be readily broken on reaction with an acid to give a compound of formula II. Thus, the term "precursor" embraces compounds which are capable of forming compounds of formula II *in situ* on reaction with an acid according to the process of the present invention and which can then be converted into compounds of formula I. without isolation of the intermediate compound of formula II.

Throughout this specification, any alkyl group, unless otherwise specified, may be linear or branched and may contain up to 12, preferably up to 6, and especially up to 4, carbon atoms. Preferred alkyl groups are methyl, ethyl; propyl and butyl. When an alkyl moiety forms part of another group, for example the alkyl moiety of an aralkyl group, it is preferred that it contains up to 6, especially up to 4, carbon atoms. Preferred alkyl moieties are methyl and ethyl.

An aryl group may be any aromatic monocylic or polycyclic hydrocarbon group and may contain from 6 to 24, preferably 6 to 18, more preferably 6 to 16, and especially 6 to 14, carbon atoms. Preferred aryl groups include phenyl, naphthyl, anthryl, phenanthryl and pyryl groups, especially a phenyl or naphthyl, and particularly a phenyl, group. When an aryl moiety forms part of another group, for example the aryl moiety of an aralkyl group, it is preferred that it is a phenyl, naphthyl, anthryl, phenanthryl or pyryl, especially a phenyl or naphthyl, and particularly a phenyl, moiety.

An aralkyl group may be any alkyl group substituted by an aryl group. A preferred aralkyl group contains from 7 to 16, especially 7 to 10, carbon atoms, a particularly preferred aralkyl group being a benzyl group.

When any of the foregoing substituents are designated as being optionally substituted, the substituent groups which are optionally present may be any one or more of those customarily employed in the modification of compounds to influence their structure/activity, stability, bioavailability or other property. Specific examples of such optional substituents include, for example, halogen atoms, nitro, cyano, hydroxyl, cycloalkyl, alkyl, haloalkyl, cycloalkyloxy, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, arylsulphinyl, arylsulphonyl, arylsulphonato, carbamoyl and alkylamido groups. When any of the foregoing substituents represents or contains an alkyl substituent group, this may be linear or branched and may contain up to 12, preferably up to 6, and especially up to 4, carbon atoms. A cycloalkyl group may contain from 3 to 8, preferably from 3 to 6, carbon atoms. An aryl group or moiety may contain from 6 to 10 carbon atoms, phenyl groups being especially preferred. A halogen atom may be a fluorine, chlorine, bromine or iodine atom and any group which contains a halo moiety, such as a haloalkyl group, may thus contain any one or more of these halogen atoms.

Although the group Ar is defined above as representing an aryl group substituted by at least one electron-donating group, it will be apparent that the group Ar may also be substituted by one or more other substituents at any of the remaining vacant substitution sites. Such additional substituents may be selected from the list of optional substituents given above. Preferred additional substituents include alkyl, hydroxyl and alkoxy groups, especially C₁₋₄ alkyl, hydroxyl and C₁₋₄ alkoxy groups. Of these, methyl, tert-butyl, hydroxyl and methoxy groups, especially tert-butyl groups, are particularly preferred.

Preferably, Ar represents a C₆₋₁₀ aryl, especially a phenyl or naphthyl, group each substituted by at least one electron-donating group.

It is particularly preferred that Ar represents a phenyl group substituted by at least one electron-donating group.

Preferably, the or each electron-donating group in the compound of formula II is located in the ortho or para position relative to the group -OR When two electron-donating groups are present, it is preferred that one of these is located in the ortho position relative to the group -OR and the other is located in the other ortho position or the para position relative to the group -OR.

An electron-donating group is any atom or group which enhances the availability of electrons about the aromatic ring to which it is attached. Typical electron-donating groups thus include substituents which have an unshared pair of electrons, especially on the atom of the substituent which is adjacent to the ring, which can stabilise intermediates in the reaction by interacting with the n electrons of the ring in an electron-donating resonance effect. Electron-donating groups also include substituents which are capable of polarising the bonding electrons of the ring thus giving rise to an electron-donating polar or polarisability effect.

Preferably, electron-donating groups are selected from the group consisting of halogen, alkyl, aryl, hydroxyl, alkoxy, aralkyloxy, aryloxy, acyloxy, alkylthio, aralkylthio, arylthio, amino, alkylamino, dialkylamino and acylamino groups. In this context, an acyl group is an alkylcarbonyl, aralkylcarbonyl or arylcarbonyl group. It is particularly preferred that the or each electron-donating group is selected from the group consisting of alkyl, hydroxyl, alkoxy, amino, alkylamino and dialkylamino groups, especially C₁₋₄ alkyl, hydroxyl, C₁₋₄ alkoxy, amino, C₁₋₄ alkylamino and di-(C₁₋₄ alkyl)amino groups. More preferably, the electron-donating group or one of the electron-donating groups is a hydroxyl group.

It is preferred that R is an optionally substituted alkyl or aralkyl, especially a C₁₋₄ alkyl or C₇₋₁₀ aralkyl, group. More preferably, R is a methyl or benzyl, especially a methyl, group.

It has been found that the commercial antioxidant BHA (butylated hydroxyanisole) can be converted to the commercial antioxidant TBHQ (tert-butyl-hydroquinone) using the process of the present invention. This is particularly advantageous since TBHQ is more expensive than BHA. Moreover, BHA and TBHQ are currently produced commercially by different synthetic pathways and the commercial advantages of a common synthetic route to both compounds will therefore be readily apparent.

Commercially available BHA is a mixture of 2-tert-butyl-4-methoxyphenol(3-tert-butyl-4-hydroxyanisole) and 3-tert-butyl-4-methoxyphenol(2-tert-butyl-4-hydroxy-anisole) and may therefore be represented by the following formula

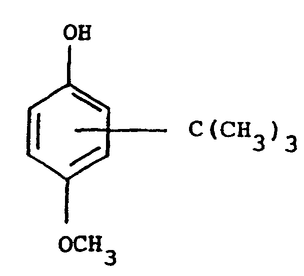

It is therefore preferred that the compound of formula II is 2-tert-butyl-4-methoxyphenol(3-tert-butyl-4-hydroxyanisole), 3-tert-butyl-4-methoxyphenol(2-tert-butyl-4-hydroxyanisole) or a mixture thereof (BHA).

It is also envisaged that the process of the present invention could be useful in the synthesis of other commercial antioxidants which contain aryl groups substituted by at least one hydroxyl group, particularly antioxidants containing a naphthol or, especially, a phenol group. Specific commercial antioxidants in this respect include BHA, BHT (butylated hydroxytoluene or 2,6-di-tert-butyl-4-methylphenol) and the gallates, particularly the alkyl gallates (alkyl esters of 3,4,5-trihydroxybenzoic acid) and, especially, the ethyl, propyl, octyl and dodecyl gallates. The compound of formula II may therefore have one of the following formulae:- 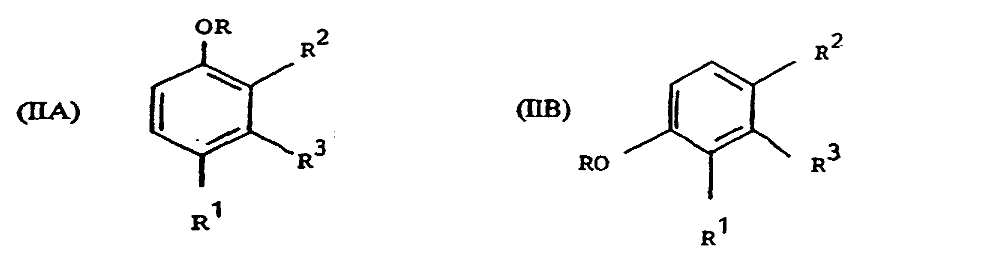 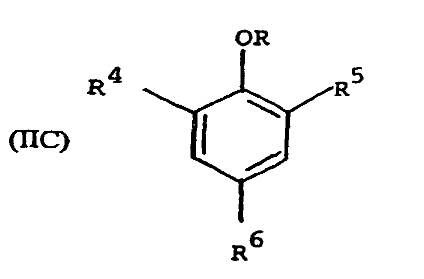 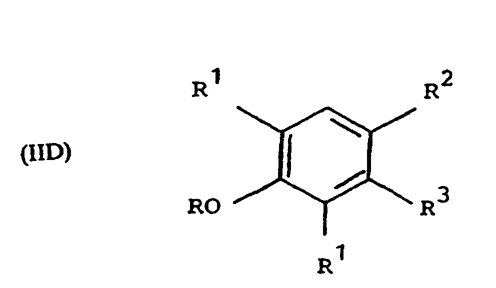 in which R, R¹, R² and R³ are as previously defined and at least one of R⁴, R⁵ and R⁶, is an electron-donating group as previously defined.

In formulae IIA and IIB, it is preferred that R is an alkyl, especially a methyl, or an aralkyl, especially a benzyl, group and R¹ is a hydroxyl or methoxy group. It is also preferred that one of R² and R³ is an alkyl, especially a tert-butyl, group and that the other of R² and R³ is a hydrogen atom. It is particularly preferred that the compound of formula IIA is BHA or a precursor of BHA which is capable of forming TBHQ when subjected to the process of the present invention. In another preferred embodiment, the compound of formula IIA is capable of forming BHA when subjected to the process of the present invention. Thus, preferably, the compound of formula IIA is a compound in which R is an alkyl or aralkyl, especially a methyl or benzyl, group, R¹ is an alkoxy or aralkoxy, especially a methoxy or benzyloxy, group, one of R² and R³ is a tert-butyl group and the other of R² and R³ is a hydrogen atom and the reaction is interrupted at an intermediate stage and the product of formula I isolated to give BHA.

In formula IIC, it is preferred that R is an alkyl, especially a methyl, or an aralkyl, especially a benzyl, group. Preferably, R⁴, R⁵ and R⁶ each independently represent an alkyl, especially a C₁₋₄ alkyl group. More preferably, R⁴ and R⁵ each represent a tert-butyl group and R⁶ represents a methyl group. It is particularly preferred that the compound of formula IIC is capable of forming BHT when subjected to the process of the present invention.

In formula (IID), is preferred that R is an alkyl, especially a methyl, or an aralkyl, especially a benzyl, group. It is also preferred that each group R¹ is a hydroxyl group.

The acid utilised in the process of the present invention must comprise at least one short chain (C₁₋₆) alkanoic acid in which the alkyl moiety is optionally substituted by a halogen atom. However, the acid may comprise a short chain alkanoic acid, a mixture of two or more short chain alkanoic acids or a mixture of one or more short chain alkanoic acids and one or more strong acids, such as mineral acids: If a mixture of one or more short chain alkanoic acids and one or more mineral acids is used, it is preferred that the or each mineral acid forms only a minor part of the total acid mixture. Preferably, the total quantity of mineral acid(s) present will not exceed 20%, more preferably 10%, of the total acid mixture.

The short chain alkanoic acid is preferably a carboxylic acid which contains from 1 to 4 carbon atoms. Most preferably, the short chain alkanoic acid is a C₁₋₃ alkanoic acid, especially methanoic acid. The alkyl moiety of the short chain alkanoic acid. may be substituted by a halogen atom as defined above. However, it is preferred that the alkyl moiety is unsubstituted.

Short chain alkanoic acids are commercially available in various grades of purity. In some circumstances, it may be advantageous to use a substantially pure, that is, 98-100% alkanoic acid. However, it is preferred that the alkanoic acid contains some water. In this respect, 85% methanoic acid is particularly preferred.

The reaction temperature selected will depend on the compound of formula II and the short chain alkanoic acid which are to be used. However, a suitable reaction temperature range is from ambient temperature, for instance, 0° to 30°C, to the reflux temperature of the reaction mixture. In general, the higher the temperature, the faster the reaction proceeds.

Antioxidants of the type described above are utilised in silage aids for the acidic preservation of organic by-products such as fish waste, slaughter waste, poultry waste and food waste in order to protect such products from oxidation. Such silage aids also include at least one acid which is used to treat the raw material to obtain the optimum pH (3:5-4.5) with regard to enzymatic hydrolysis. It is therefore a further advantage of the process of the present invention that an antioxidant such as TBHQ can be produced in an acid solution which can be used without further processing, for instance, as a silage aid in the silage industry. In this respect, it is particularly preferred that the acid used in the process is methanoic acid, especially 85% methanoic acid. Silage aids comprising at least one antioxidant and at least one short chain carboxylic acid form the subject of co-pending International Patent Application No. PCT/NO00/00079 the contents of which are hereby incorporated by reference. However, if the end-product of formula I is to be used in another application, the acid can easily be removed by conventional means.

The invention is further illustrated by the following examples.

### Example 1

### Preparation of TBHQ

BHA (1.5g) was dissolved in 85% methanoic acid (100 ml) and the solution was heated with continuous stirring. After refluxing for about 24 hours, analysis by ¹H-NMR spectroscopy revealed that ether cleavage had taken place to produce a solution of TBHQ in 85% methanoic acid.

### Example 2

### Preparation of TBHQ

BHA was dissolved in 85% methanoic acid to give a concentration of 0.75% by weight BHA and the solution was then stored at room temperature (25°C). Analysis by HPLC and ¹H-NMR spectroscopy at regular time intervals gave the following results:-

| Elapsed time | % by weight BHA |
|---|---|
| 2 weeks | 0.72 |
| 5 weeks | 0.69 |
| 10 weeks | 0.15 |
| 22 weeks | 0.10 |

Conversion of BHA to TBHQ was confirmed by ¹H-NMR spectroscopy.

### Example 3 : (Not in accordance with the invention)

### Preparation or hydroquinone

Hydroquinone monobenzyl ether (1.5g) was dissolved in 85% methanoic acid (100 ml) and the solution was heated with continous stirring. After refluxing for about 3 hours, analysis by ¹H-NMR spectroscopy revealed that ether cleavage had taken place to produce a solution of hydroquinone in 85% methanoic acid.

## Claims

1. A process for the preparation of a compound of the general formula
ArOH (I)
in which Ar represents a group of the general formula 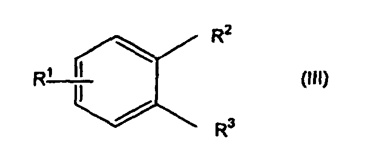 in which R¹ represents an electron-donating group, one of R² and R³ independently represents a hydrogen atom or an optionally substituted alkyl group and the other of R² and R³ represents an optionally substituted alkyl group, or R² and R³ together with the interjacent carbon atoms form an optionally substituted aryl group, which comprises reacting a compound of the general formula
ArOR (II)
or a compound which is capable of forming a compound of formula II in situ on reaction with a C₁₋₆ alkanoic acid in which the alkyl moiety is optionally substituted by a halogen atom, in which Ar is as defined above and R is an optionally substituted alkyl, aryl or aralkyl group, with an acid comprising at least one C₁₋₆ alkanoic acid in which the alkyl moiety is optionally substituted by a halogen atom.

2. A process according to claim 1 in which Ar represents a C₈₋₁₀ aryl group substituted by at least one electron-donating group.

3. A process according to any one of the preceding claims in which Ar represents a phenyl group substituted by at least one electron-donating group.

4. A process according to any one of the preceding claims in which the or each electron-donating group in the compound of formula II is located in the ortho or para position relative to the group -OR.

5. A process according to any one of the preceding claims in which the or each electron-donating group is selected from the group consisting of alkyl, hydroxyl, alkoxy, amino, alkylamino and dialkylamino groups.

6. A process according to any one of the preceding claims in which the electron-donating group or one of the electron-donating groups is a hydroxyl group.

7. A process according to any one of the preceding claims in which R is an optionally substituted C₁₋₄ alkyl or C₇₋₁₀ aralkyl group.

8. A process according to any one of the preceding claims in which R is a methyl group.

9. A process according to any one of the preceding claims in which the compound of formula II is 2-tert-butyl-4-methoxyphenol (3-tert-butyl-4-hydroxyanisole), 3-tert-butyl-4-methoxyphenol (2-tert-butyl-4-hydroxyanisote) or a mixture thereof (BHA).

10. A process according to any one of the preceding claims in which the acid comprises a C₁₋₆ alkanoic acid in which the alkyl moiety is optionally substituted by a halogen atom, a mixture of two or more C₁₋₆ alkanoic acids in which the alkyl moiety is optionally substituted by a halogen atom, or a mixture of one or more C₁₋₆ alkanoic acids in which the alkyl moiety is optionally substituted by a halogen atom and one or more mineral acids.

11. A process according to any one of the preceding claims in which the or each C₁₋₆ alkanoic acid is a C₁₋₃ alkanoic acid.

12. A process according to any one of the preceding claims in which the acid is methanoic acid.

## Patentansprüche

1. Verfahren zur Herstellung einer Verbindung der allgemeinen Formel
ArOH (I)
wobei Ar eine Gruppe der folgenden allgemeinen Formel repräsentiert 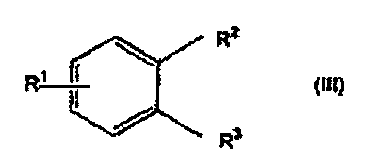 wobei R¹ eine elektronenabgebende Gruppe repräsentiert, R² oder R³ unabhängig ein Wasserstoffatom oder eine optional substituierte Alkylgruppe repräsentiert und das jeweils andere von R² und R³ eine optional substituierte Alkylgruppe repräsentiert oder R² und R³ zusammen mit den dazwischen liegenden Kohlenstoffatomen eine optional substituierte Arylgruppe bilden, umfassend das Reagieren einer Verbindung der allgemeinen Formel
ArOR (II)
oder einer Verbindung, die in situ eine Verbindung der Formel II auf eine Reaktion mit einer C₁₋₆ Alkansäure hin bilden kann, in der der Alkylanteil optional substituiert ist durch ein Halogenatom, wobei Ar der obigen Definition entspricht und R eine optional substituierte Alkyl-, Aryl- oder Aralkylgruppe ist, mit einer Säure, umfassend wenigstens eine C₁₋₆ Alkansäure, in der der Alkylanteil optional durch ein Halogenatom substituiert ist.

2. Verfahren nach Anspruch 1, wobei Ar eine C₆₋₁₀ Arylgruppe repräsentiert, die substituiert ist durch wenigstens eine elektronenabgebende Gruppe.

3. Verfahren nach einem der vorherigen Ansprüche, wobei Ar eine Phenylgruppe repräsentiert, die durch wenigstens eine elektronenabgebende Gruppe substituiert ist.

4. Verfahren nach einem der vorherigen Ansprüche, wobei die oder jede elektronenabgebende Gruppe in der Verbindung der Formel II in der ortho- oder para-Position relativ zur Gruppe -OR vorliegt.

5. Verfahren nach einem der vorherigen Ansprüche, wobei die oder jede elektronenabgebende Gruppe ausgewählt wird aus der Gruppe bestehend aus Alkyl-, Hydroxyl-, Alkoxy-, Amino-, Alkylamino- und Dialkylaminogruppen.

6. Verfahren nach einem der vorherigen Ansprüche, wobei die elektronenabgebende Gruppe oder eine der elektronenabgebenden Gruppen eine Hydroxylgruppe ist.

7. Verfahren nach einem der vorherigen Ansprüche, wobei R eine optional substituierte C₁₋₄ Alkyl- oder C₇₋₁₀ Aralkylgruppe ist.

8. Verfahren nach einem der vorherigen Ansprüche, wobei R eine Methylgruppe ist.

9. Verfahren nach einem der vorherigen Ansprüche, wobei die Verbindung der Formel II 2-tert-Butyl-4-methoxyphenol(3-tert-butyl-4-hydroxyanisol), 3-tert-Butyl-4-methoxyphenol(2-tert-butyl-4-hydroxyanisol) oder ein Gemisch davon ist (BHA).

10. Verfahren nach einem der vorherigen Ansprüche, wobei die Säure Folgendes umfasst: eine C₁₋₆ Alkansäure, in der der Alkylanteil optional substituiert ist durch ein Halogenatom, ein Gemisch aus zwei oder mehr C₁₋₆ Alkansäuren, in denen der Alkylanteil optional substituiert ist durch ein Halogenatom, oder ein Gemisch aus einer oder mehreren C₁₋₆ Alkansäuren, in denen der Alkylanteil optional substituiert ist durch ein Halogenatom, und eine oder mehrere Mineralsäuren.

11. Verfahren nach einem der vorherigen Ansprüche, wobei die oder jede C₁₋₆ Alkansäure eine C₁₋₃ Alkansäure ist.

12. Verfahren nach einem der vorherigen Ansprüche, wobei die Säure Methansäure ist.

## Revendications

1. Un procédé pour la préparation d'un composé de la formule générale
ArOH (I)
dans laquelle Ar représente un groupe de la formule générale 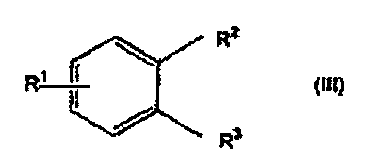 dans laquelle R¹ représente un groupe donneur d'électrons, l'un de R² et R³ représente indépendamment un atome d'hydrogène ou un groupe alkyle substitué optionnellement et l'autre de R² et R³ représente un groupe alkyle substitué optionnellement, ou R² et R³ avec les atomes de carbone intercalés forment un groupe aryle substitué optionnellement qui comprend la réaction d'un composé de la formule générale
ArOR (II)
ou d'un composé qui est capable de former un composé de la formule II in situ sur réaction avec un acide alcanoïque C₁₋₆ dans lequel la partie alkyle est substituée optionnellement par un atome d'halogène, dans lequel Ar est tel que défini ci-dessus et R est un groupe alkyle, aryle ou aralkyle substitué optionnellement, avec un acide comprenant au moins un acide alcanoïque C₁₋₆ dans lequel la partie alkyle est substituée optionnellement par un atome d'halogène.

2. Un procédé selon la revendication 1 dans lequel Ar représente un groupe aryle C₆₋₁₀ substitué par au moins un groupe donneur d'électrons.

3. Un procédé selon l'une quelconque des revendications précédentes dans lequel Ar représente un groupe phényle substitué par au moins un groupe donneur d'électrons.

4. Un procédé selon l'une quelconque des revendications précédentes dans lequel le ou chaque groupe donneur d'électrons dans le composé de la formule il est situé à la position ortho ou para par rapport au groupe -OR.

5. Un procédé selon l'une quelconque des revendications précédentes dans lequel le ou chaque groupe donneur d'électrons est sélectionné dans le groupe constitué des groupes alkyle, hydroxyle, alkoxy, amine, alkylamine et dialkylamine.

6. Un procédé selon l'une quelconque des revendications précédentes dans lequel le groupe donneur d'électrons ou l'un des groupes donneurs d'électrons est un groupe hydroxyle.

7. Un procédé selon l'une quelconque des revendications précédentes dans lequel R est un groupe alkyle C₁₋₄ ou aralkyle C₇₋₁₀ substitué optionnellement.

8. Un procédé selon l'une quelconque des revendications précédentes dans lequel R est un groupe méthyle.

9. Un procédé selon l'une quelconque des revendications précédentes dans lequel le composé de la formule II est 2-tert-butyle-4-méthoxyphénol (3-tert-butyle-4-hydroxyanisote), 3-tert-butyle-4-méthoxyphénol (2-tert-butyle-4-hydroxyanisole) ou un mélange de ceux-ci (BHA).

10. Un procédé selon l'une quelconque des revendications précédentes dans lequel l'acide comprend un acide alcanoïque C₁₋₆ dans lequel la partie alkyle est substituée optionnellement par un atome d'halogène, un mélange de deux acides alcanoïques C₁₋₆ ou plus dans lequel la partie alkyle est substituée optionnellement par un atome d'halogène ou un mélange d'un ou plusieurs acides alcanoïques C₁₋₆ dans lequel la partie alkyle est substituée optionnellement par un atome d'halogène et un ou plusieurs acides minéraux.

11. Un procédé selon l'une quelconque des revendications précédentes dans lequel le ou chaque acide alcanoïque C₁₋₆ est un acide alcanoïque C₁₋₃.

12. Un procédé selon l'une quelconque des revendications précédentes dans lequel l'acide est de l'acide méthanoïque.
